(19) Europäisches Patentamt
European Patent Office
Office européen des brevets

(11) Publication number: 0 013 467
A1

(12) EUROPEAN PATENT APPLICATION

(21) Application number: 79300066.2

(22) Date of filing: 16.01.79

(51) Int. Cl.³: C 07 D 307/36
B 01 J 31/26

(43) Date of publication of application:
23.07.80 Bulletin 80/15

(84) Designated Contracting States:
BE CH DE FR GB LU NL SE

(71) Applicant: THE STANDARD OIL COMPANY
Midland Building
Cleveland, Ohio 44115(US)

(72) Inventor: Herrington, Daniel Robert
13415 Shady Lane Chesterland
Ohio 44026 Geauga County(US)

(72) Inventor: Schwerko, Albert Peter
36695 Timberland Drive Solon
Ohio 44139 Cuyahoga County(US)

(74) Representative: Stagg, Diana Christine et al,
ELKINGTON & FIFE High Holborn House 52-54 High Holborn
London WC1V 6SH(GB)

(54) **Process for the manufacture of furan compounds.**

(57) A process for the conversion of an acyclic conjugated diolefinic hydrocarbon containing from 4 to 10 carbon atoms, for example butadiene, to furan or an alkyl-substituted furan, which comprises reacting the conjugated diolefin with molecular oxygen in the liquid phase characterised in that the reaction is carried out in an inert organic solvent in the presence of a catalyst having the composition:

$[R_xM(L)_y]_z$

in which

R represents an organic ligand which is an alkyl, aryl, alkene, diene, triene, or alkyne radical containing from 1 to 8 carbon atoms;

L represents a ligand which is carbon monoxide and a halogen;

M represents a transition metal or mixture of transition metals from Groups IVB, VB, VIB, VIIB and VIII of the Periodic classification of elements;

x is 0 to 2;

y is 0 to 6;

x + y is 1 to 6; and

z is 1 to 6.

The reaction is preferably carried out at a temperature of from 20 to 200°C.

EP 0 013 467 A1

Title

PROCESS FOR THE MANUFACTURE OF FURAN COMPOUNDS

The present invention relates to a process for the manufacture of furan compounds by the direct oxidation of conjugated diolefins with air or oxygen in the liquid phase in the presence of a transition metal catalyst system.

Present processes for the direct oxidation of diolefins to furan compounds are primarily vapour phase processes which are generally characterized by low conversion and poor selectivity. These disadvantages result from the instability of furan compounds at high temperatures in the presence of oxygen, which leads to the formation of resinous compounds, charring and uncontrolled polymerization. The liquid phase process of the present invention eliminates these disadvantages by operating at moderate temperatures.

Although several liquid phase processes are known for the production of furan compounds, they involve the use of oxygenated compounds as starting materials. For example, U.S. Patent No. 3,932,468, and U.S. Patent No. 3,996,248, relate to the rearrangement of butadiene monoxide, and U.S. Patent No. 3,933,861, relates to the reaction of an alkene and an alkene oxide to yield substituted furans. Both of these processes require oxygenated starting materials,

whereas in the present invention, furan compounds are obtained by the direct oxidation of the conjugated diolefin.

While Japanese Patent No. 77 77,049 discloses a process for the oxidation of butadiene to furan in an aqueous acidic medium, the process of the present invention is distinguished from this process in that the present process is conducted in an organic solvent medium in which the catalyst and furan products are more stable.

According to the process of the present invention, an acyclic conjugated diolefin containing from 4 to 10 carbon atoms is converted to furan or an alkyl-substituted furan compound by the direct oxidation of the diolefin with molecular oxygen in a liquid phase reaction. The reaction is carried out in a non-aqueous reaction medium in the presence of a transition metal organo-metallic catalyst complex.

The liquid phase oxidation reaction of this invention is a free radical reaction, and these reactions appear to be initiated by means of the formation of an initial free radical. This initial free radical may generate the desired product (furan) directly, or proceed to form other radical intermediates which can yield either furan, other oxygenated products, such as a diolefin monoxide, 2,5-dihydrofuran, crotonaldehyde, or oligomers and/or polymers.

The role of the catalyst of this invention is to react with the initial key radical intermediates, converting them directly to furan products before deleterious by-products can be produced. It is this selective catalytic behavior, coupled with specific reaction conditions herein defined, that result in the enhanced selectivity of the oxidation of the

diolefins to the desired furan compounds.

The invention therefore provides a process for the conversion of an acyclic conjugated diolefinic hydrocarbon containing from 4 to 10 carbon atoms to furan or an alkyl-substituted furan which comprises reacting the conjugated diolefin with molecular oxygen in the liquid phase characterised in that the reaction is carried out in an inert organic solvent in the presence of a catalyst having the composition:

$$[R_xM\ (L)_y]_z$$

in which R represents an organic ligand which is an alkyl, aryl, alkene, diene, triene, or alkyne radical containing from 1 to 8 carbon atoms;

L represents a ligand which is carbon monoxide and a halogen;

M represents a transition metal or mixture of transition metals from Groups IVB, VB, VIB, VIIB and VIII of the Periodic classification of elements;

x is 0 to 2,

y is 0 to 6;

x + y is 1 to 6 and

z is 1 to 6.

Suitable feeds in this invention for conversion to furan compounds comprise acyclic alkadienes having from 4 to 10 carbon atoms. Examples include butadiene-1,3, pentadiene-1,3, isoprene, hexadiene-1,3, and decadiene-1,3, and mixtures thereof. The acyclic alkadienes having from 4 to 5 carbon atoms are preferred in this process. The furan compounds produced by the process of the present invention have the formula:

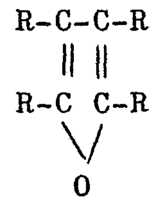

in which each R which may be the same or different represents hydrogen or an alkyl radical having from 1 to 6 carbon atoms, the total carbon atoms in the R radicals being in the range of 0 to 6. Representative compounds include furan, 2-methylfuran, 3-methylfuran, 2,5-diethylfuran, 2-n-hexylfuran, 2-isopropyl-3-methylfuran, 3,4-n-dipropylfuran and 3-methyl-4-n-butylfuran.

The catalysts for use in the process of this invention are organo-metallic complexes or salts of the metals of Groups IVB, VB, VIB, VIIB or VIII of the Periodic classification of elements. These complexes have the general formula:

$$[R_xM\ (L)_y]_z$$

in which R represents an organic ligand which is an alkyl, aryl, alkene, diene, triene or alkyne radical containing from 1 to 8 carbon atoms;

L represents a ligand which is carbon monoxide or a halogen;

M represents a transition metal or a mixture of transition metals from the Groups IVB, VB, VIB, VIIB and VIII of the Periodic classification of elements;

x is 0 to 2;

y is 0 to 6;

x + y is 1 to 6;

and z is 1 to 6.

Specific examples of suitable catalysts include $OsCl_3$, $Os_3(CO)_{12}$, $[CpMo(CO)_3]_2$ (Cp = cyclopentadienyl radical)

$CpV(CO)_4$, $CpTiCl_2$, $CpMn(CO)_3$, $(Cp)_2Fe$, $Mo(CO)_6$, $[CpFe(CO_2]_2$, $(C_4H_6)Fe(CO)_3$, $Co_2(CO)_8$, $Ru_3(CO)_{12}$, $Rh_6(CO)_{16}$ and $W(CO)_6$.

While these complexes and salts are effective catalysts in their own right, it may also be advantageous to utilize certain promotors for these catalysts as defined by the general formula:

$$A\ R_m\ X_n$$

in which A represents mercury, thallium, indium, or a Group IV A element such as silicon, germanium, tin or lead;

R represents a hydride, alkyl, aryl or amine group;

X represents an anion of a mineral acid or carboxylic acid;

m is 0-4;

n is 0-4

and m + n is 1 to 4.

Specific examples of these types of promoters include such compounds as $Hg(C_2H_3O_2)_2$, $SnCl_2$, $(C_2H_5)_2SnCl_2$, $SnCl_4$, $(CH_3)_3SnN(CH_3)_2$, $GeI_2$, $(n\text{-}C_4H_9)_3GeI$, $(\sigma\text{-}C_5H_5)Ge(CH_3)_3$, $(C_2H_5)_3\ PbCl$, $(CH_3)_3\ SiH$, or $SiH_3I$.

When promoters are employed for the catalysts of this invention, they may be added to the reaction mixture as separate species or they may be reacted with the catalyst to give a separate chemical compound which can be isolated and purified prior to its use as a catalytic agent. Representative examples of compounds formed by reactions occurring between the catalyst and the promoter include: $ClHgFe(Cp)_2$, $Hg[Co(CO)_4]_2$, $Cl_2Sn[Fe(CO)_2Cp]_2$, $I_2Ge[Co(CO)_4]_2$, $[(C_2H_5)_3Pb]_2Fe(CO)_4$, $H_3SiCo(CO)_4$, $Cl(CH_3)_2Sn[Mn(CO)_5]$,

and $[Cp(CO)_3Mo-Sn(CH_3)_2-Mn(CO)_5]$.

The promoter compounds of the catalyst system are preferably used in molar ratios of from 0.25 to 2.0 moles of promoter per mole of the transition metal catalyst. Particularly preferred molar ratios of promoter compound to transition metal catalyst are from 0.5:1 to 2:1. The catalysts used in the process of this invention, with or without promoters may be dissolved in the reaction medium as homogeneous catalysts, slurried in the reaction medium as insoluble, unsupported heterogeneous catalysts, or in some cases where advantageous, they may be supported on carriers such as silica, alumina, or polymeric materials and slurried in the reaction medium. It is however preferred, that the catalyst system is a homogeneous system where the catalyst is soluble in the reaction solvent. The concentration of the catalyst in the solvent medium may range from $10^{-6}$ to 10.0 moles/liter. A catalyst concentration of from about $10^{-5}$ to 1.0 moles/liter is preferably used.

The reaction medium suitable for the process of this invention is an essentially inert, non-coordinating or weakly coordinating organic solvent having a boiling point significantly higher than the boiling points of the feed or the products obtained. Solvents with boiling points of from 130° to 225°C are especially preferred. Those solvents having an absence of abstractable hydrogens which could lead to oxidation of the solvent or the binding of the active sites of the metal or metals in the catalyst, thereby deactivating the catalyst, are also preferred. Examples of suitable solvents include paraffinic hydrocarbons, aromatic hydrocarbons, chlorinated hydrocarbons, and nitrile aromatics such as heptanes and decanes, toluene and the xylenes; chlorobenzene, chloroform and carbon tetrachloride; and benzonitrile; with chlorobenzene being the most preferred. Substituted furans such as alkylfurans or 2,3-benzofuran may also serve as suitable solvents in some cases.

The oxidation reaction of the present invention is very sensitive to reaction conditions and it is an essential feature of the invention that the reaction is carried out under conditions which maximize selectivity. The reaction may be carried out at a temperature in the range of from 20° to 200°C, and preferably at a temperature in the range of from about 50° to 130°C. Temperatures above this range result in the formation of additional oxidation products such as crotonaldehyde and increase the formation of undesirable polymer.

The reaction pressure may range from 1 to 20 atmospheres, and preferably from 1 to 10 atmospheres. The partial pressure of oxygen is of particular importance to the selectivity of the reaction, and oxygen pressures of from 0.5 to 5 atmospheres, especially from 1 to 3 atmospheres, are preferably employed.

Another critical reaction variable affecting the selectivity of the reaction is the ratio of diolefin to oxygen. While the molar ratio of diolefin to oxygen may vary from 0.001 to 100.0, a ratio of from 0.33 to 5.0 is preferred.

Where the reaction is carried out in a sealed reaction vessel, the reaction times may range from 0.5 to 10 hours and a reaction time of from 1.0 to 4 hours is preferable. Continuous operation in which the reaction mixture is maintained at constant temperature and pressure is also within the scope of the present invention. Under such conditions, the diolefin and air or oxygen are fed continuously to the

reactor while volatile products and the unreacted feed are removed continuously. The volatile products can be collected and the unreacted feed recycled to the reactor.

The reactor vessel may be constructed from stainless steel, or in certain instances the reaction vessel may be lined with glass, quartz or a stable resinous material in order to minimise side reactions between reaction intermediates and the walls of the reaction vessel.

The invention will now be further described with reference to the following Examples.

EXAMPLES 1-12

The oxidation of butadiene to furan in the presence of a variety of promoted and unpromoted transition metal catalyst complexes was conducted in a series of experiments according to the following procedure:

An amount of catalyst required to give a concentration of 1 x $10^{-4}$ moles of catalyst in the reaction solvent was weighed into a stainless steel reaction tube (180 mm long x 9.5 mm diameter) equipped with a stainless steel ball valve and septum cap. The tube was evacuated and charged with a mixture of butadiene and oxygen in a 1:1 molar ratio at an initial oxygen pressure of 2.2 atmospheres. Four millilitres of chlorobenzene solvent was introduced into the tube with a metering pump. The tube and its contents were heated to a temperature of 110°C in a heating block for a period of two hours. At the end of this time period, the tube was quickly cooled to room temperature and the reaction mixture analysed by gas chromatography.

The percent conversion of the butadiene and the percent selectivity to furan based on the percent of butadiene converted that were obtained in Examples 1 to

12 are summarized in Table I below.

TABLE I

| Example | Catalyst | % Total Conversion | % Selectivity to Furan |
|---|---|---|---|
| 1 | $(Cp)_2Fe$ | 2.9 | 99.0 |
| 2 | $(Cp)_2Fe/SnCl_2$ | 10.2 | 81.1 |
| 3 | $Mo(CO)_6$ | 1.4 | 97.7 |
| 4 | $Mo(CO)_6/Hg(C_2H_3O_2)_2$ | 16.7 | 65.4 |
| 5 | $CpV(CO)_4$ | 9.4 | 82.2 |
| 6 | $CpTi_2Cl_2$ | 13.3 | 77.5 |
| 7 | $[CpMo(CO)_3]_2$ | 17.6 | 68.2 |
| 8 | $Os_3(CO)_{12}$* | 18.2 | 92.0 |
| 9 | $Os_3(CO)_{12}/SnCl_2$ | 14.2 | 71.6 |
| 10 | $OsCl_3$ | 20.5 | 57.4 |
| 11 | $Ru_3(CO)_{12}$ | 0.1 | 100.0 |
| 12 | $Ru_3(CO)_{12}/(n-C_4H_9)_3GeI$ | 13.2 | 99.0 |

(Cp = cyclopentadiene)

*Reaction conducted in a resin coated stainless steel reactor.

CLAIMS:

1. A process for the conversion of an acyclic conjugated diolefinic hydrocarbon containing from 4 to 10 carbon atoms to furan or an alkyl-substituted furan which comprises reacting the conjugated diolefin with molecular oxygen in the liquid phase characterised in that the reaction is carried out in an inert organic solvent in the presence of a catalyst having the composition:

$$[R_xM\ (L)_y]_z$$

in which R represents an organic ligand which is an alkyl, aryl, alkene, diene, triene, or alkyne radical containing from 1 to 8 carbon atoms;

L represents a ligand which is carbon monoxide and a halogen;

M represents a transition metal or mixture of transition metals from Groups IVB, VB, VIB, VIIB and VIII of the Periodic classification of elements;

x is 0 to 2;

y is 0 to 6;

x + y is 1 to 6; and

z is 1 to 6.

2. A process as claimed in Claim 1, characterised in that the catalyst is promoted with a compound having the formula:

$$A\ R_m\ X_n$$

in which A represents mercury, thallium, indium, silicon, germanium, tin lead;

R represents a hydride, alkyl, aryl or amine radical;

X represents an anion of a mineral acid or a carboxylic acid;

m and n each are numbers from 0 to 4

and $m + n$ is 1 to 4.

3. A process as claimed in Claim 2, characterised in that the promoter is used in a molar ratio of from 0.25 to 2.0 moles per mole of the transition metal catalyst.

4. A process as claimed in any of Claims 1 to 3, characterised in that the reaction is carried out at a temperature of from 20° to 200°C.

5. A process as claimed in any of Claims 1 to 4, characterised in that the molar ratio of diolefin to oxygen is from 0.001 to 100.0.

6. A process as claimed in any of Claims 1 to 5, characterised in that the reaction is carried out in an inert organic solvent having a boiling point in the range of from 130 to 225°C.

7. A process as claimed in Claim 6, characterised in that the solvent is a paraffinic hydrocarbon; an aromatic hydrocarbon; a chlorinated hydrocarbon; a nitrile aromatic, or an alkyl or aryl-substituted furan.

8. A process as claimed in Claim 7, characterised in that the solvent is chlorobenzene.

9. A process as claimed in any of Claims 1 to 8, characterised in that the catalyst is soluble in the reaction solvent; or is slurried in the reaction solvent.

10. A process as claimed in any of Claims 1 to 9, characterised in that the diolefin is butadiene.

0013467

Application number

European Patent Office

# EUROPEAN SEARCH REPORT

EP 79 300 066.2

## DOCUMENTS CONSIDERED TO BE RELEVANT

| Category | Citation of document with indication, where appropriate, of relevant passages | Relevant to claim |
|---|---|---|
| A | DE - B - 1 816 697 (PHILLIPS PETROLEUM) -- | |
| A | DE - B - 1 281 421 (UNION CARBIDE CORP.) -- | |
| A | US - A - 4 080 312 (PHILLIPS PETROLEUM) -- | |
| A | US - A - 3 928 389 (PHILLIPS PETROLEUM) -- | |
| A | US - A - 3 238 225 (PETRO-TEX) -- | |
| A | US - A - 2 900 396 (E.I. DU PONT DE NEMOURS) ---- | |

CLASSIFICATION OF THE APPLICATION (Int. Cl.3)

C 07 D 307/36
B 01 J 31/26

TECHNICAL FIELDS SEARCHED (Int.Cl.3)

B 01 J 31/26
B 01 J 31/28
C 07 D 307/00
C 07 D 307/02
C 07 D 307/34
C 07 D 307/36

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| [illegible] | 06-09-1979 | KAPTEYN |